(19) Europäisches Patentamt
European Patent Office
Office européen des brevets

(11) Publication number: **0 177 574 B1**

(12) **EUROPEAN PATENT SPECIFICATION**

(45) Date of publication of patent specification: **20.12.89**

(51) Int. Cl.$^4$: **C 07 C 101/20**

(21) Application number: **85901831.9**

(22) Date of filing: **28.03.85**

(86) International application number: **PCT/US85/00512**

(87) International publication number: **WO 85/04396 10.10.85 Gazette 85/22**

(54) **AMINOCARNITINES.**

(30) Priority: **02.04.84 US 596180**

(43) Date of publication of application: **16.04.86 Bulletin 86/16**

(45) Publication of the grant of the patent: **20.12.89 Bulletin 89/51**

(84) Designated Contracting States: **AT BE CH DE FR GB IT LI NL**

(56) References cited:
EP-A-0 080 695
EP-A-0 127 098
FR-A-1 442 318
GB-A-1 093 937
JP-A-28 000 092
US-A-3 185 729

**The file contains technical information submitted after the application was filed and not included in this specification**

(73) Proprietor: **CORNELL RESEARCH FOUNDATION, INC.**
**East Hill Plaza**
**Ithaca, N.Y. 14850 (US)**

(72) Inventor: **GRIFFITH, Owen, W.**
**430 East 63rd Street**
**Apartment No. 6H New York, NY 10021 (US)**

(74) Representative: **Kraus, Walter, Dr. et al**
**Patentanwälte Kraus, Weisert & Partner**
**Thomas-Wimmer-Ring 15**
**D-8000 München 22 (DE)**

(56) References cited:
**Chemical Abstracts, vol. 64, 1966, Abstract no. 1970F, Marly "Carnitinamide derivatives", Belg. 659,194, Published 3 August 1965**

**Chemical Abstracts, vol. 64, 1960 Abstract no. 11314e, Marly"Carnitinamide d-camphorate", Belg. 660039, Published 23 August 1965**

**Chemical Abstracts, vol. 59, 1963, no. 11,660g; Takeda, "Carnitineamide" Japan 23, Published 9 January 1963**

**EP 0 177 574 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

Chase, J.F.A.., et al., "Specific Alkylation of a Histidine Residue in Carnitine Acetyltransferase by Bromoacetyl-L-Carnitine", Biochem. J. 116, 713-720 (1970)

Fritz, I.B., et al., "Properties of Partially Purified Carnitine Acetyltransferase", The Journal of Biological Chemistry, 238(7), 2509-2517 (7/63)

Fritz, I.B., et al., "Carnitine Acetyltransferase II. Inhibition by Carnitine Analogues and by Sulfhyderyl Reagents", The Journal of Biological Chemistry, 240(5), 2188-2192 (5/65)

Gilbert, R.J., et al., "Bromoacetyl-L-Carnitine: Biochemical and Antitrysanosomal Actions Against Trypanosoma Brucei Brucei", Biochemical Pharmacology, 32(22), 3447-3451 (1983)

Kallai-Sanfacon, M.A., et al., "Efect of AY-25,712 and Other Lipid-Lowering Agents on Liver Catalase and Liver Carnitine Acetyltransferase in Rats (41658)", Proceedings of the Society for Experimental Biology and Medicine, 173, pp. 367-371 (1983)

Sire, O., et al., "Carnitine palmitoyltransferase I", European Journal of Biochemistry, 136, pp. 371-375 (1983)

Tutwiler G., discussion in Carnitine Biosynthesis, Metabolism, and Functions, pp. 171-173, Acedemic Press, New York, 1980

Vasudevan, V., et al., "Active-Site-Directed Inhibition of Carnitine Acetyltransferase", Archives of Biochemistry and Biophysics, 220 (1), pp. 193-199 (Jan.1983)

**Description**

The invention is directed to the preparation of carnitine analogues which in competition with acyl carnitines bind and inhibit carnitine acyltransferases.

Carnitine acetyltransferase is found in mammals but its *in vivo* role has not been definitively established. There is conjecture, however, that it allows acetyl carnitine to buffer the pool of acetyl-Coenzyme A and/or that it may be involved in intracellular transport of acetyl and other short chain acyl groups. Compounds which competitively bind to and thus inhibit carnitine acetyltransferse, are useful to investigate the *in vivo* role of carnitine acetyltransferase and to verify or disprove the conjecture.

Fritz, I. B. and Schultz, S. K. "Carnitine Acetyltransferse II. Inhibition by Carnitine Analogues and by Sulfhydryl Reagents", J. Biol. Chem., *240*, 2188—2192 (1965) investigate the carnitine acetyltransferase inhibiting power of various carnitine analogues and other compounds. They do this by using carnitine acetyltransferase to catalyze the reaction

$$\text{Acetylcarnitine+Coenzyme A} \rightleftharpoons \text{acetyl-Coenzyme A+carnitine}$$

and measuring the velocity of reaction in the presence of the tested compounds and record results in terms of $K_i'$ values where lower values indicate greater inhibiting power. The inhibitors uncovered by Fritz and Schultz are relatively weak and are subject to metabolism and thus are not suitable for the role investigation previously mentioned.

Carnitine palmitoyltransferse (CPT) has a recognized role in mammals in the following chain of reactions. Outside the mitochrondria, it catalyzes the reaction

$$\text{Long chain acetyl-Coenzyme A+carnitine} \rightleftharpoons \text{long chain acyl carnitine+Coenzyme A}$$

The long chain acyl carnitine is carried by carnitine transporter from cytoplasm into the mitochondrial matrix. Inside the mitochondria CPT catalyzes the reaction

$$\text{Long chain acyl carnitine+Coenzyme A} \rightleftharpoons \text{long chain acyl-Coenzyme A+carnitine}$$

Within the mitochrondria, the long chain acyl-Coenzyme A is catabolized to carbon dioxide and in the case of diabetics to ketones leading to ketoacidosis.

It has been suggested by G. Tutwiler in *Carnitine Biosynthesis, Metabolism and Functions,* Academic Press, N.Y., pp. 171—173 (1980), that inhibiting the fatty acid catabolism may reverse such ketoacidosis. Compounds which competively bind to and thus inhibit CPT are useful to investigate whether interruption of fatty acid catabolism does reverse ketoacidosis and are useful in the treatment of diabetes and as a substitute or supplement for insulin.

Bromoacetyl-L-carnitine has been shown *in vitro* to have a potent effect against *T. Brucei*, the causitive agent of African trypanosomiases. See Gilbert, R. J., Klein, R. A., and Johnson, P., "Bromoacetyl-L-Carnitine: Biochemical and Antitrypanosomal Actions Against *Trypansoma Brucei Brucei*", Biochem. Pharmacol. *32*, No. 22, 3447—3451 (1983). The potential of bromoacetyl-L-carnitine is limited *in vivo* because of toxicity due to release of bromine and/or bromoacetate. A more stable analog would eliminate this toxic effect.

Acetylated aminocarnitines have been described herein which resist metabolizing and are highly stable and strongly bind to corresponding carnitine acyltransferses and function as excellent competitive inhibitors thereof and thus provide excellent research tools for investigating the role of the transferases in the body i.e. for evaluating the specificity of carnitine acyltransferases. The acetylated compounds are useful to investigate the role of carnitine acetyltransferase in the body, i.e. to investigate the specificity of carnitine acetyltransferase. The long chain acyl compounds are useful to investigate the role of fatty acid catabolism in diabetes and to control ketogenesis and as a supplement or substitute for insulin to control the complications of diabetes.

Haloacylated compounds herein bind in non-reversible fashion to the corresponding carnitine acyltransferases and thus are longer lastering than the unsubstituted acyl compounds. Moreover, the haloacylated compounds are stable and thus cure the deficiencies of the bromoacylated carnitines in respect to *in vivo* treatment of trypanosomiases.

Nonacylated aminocarnitines herein are intermediates for the acylated compounds and have the potential for binding fatty acids in mammals so that they are excreted.

EP—A—0 080 695 relates to a compound of the formula

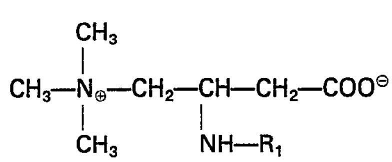

wherein $R_1$ is a hydrogen atom or an acetyl group, or a salt thereof and to a process for preparing the compound. According to the present invention compounds are prepared, wherein the amino substituent is a long chain acyl. Long chain acyl compounds are unexpectedly potent compared with the acetyl compound. As outlined above, these compounds react with carnitine acyl transferase to bind it and competitively inhibit it from participating in a reaction of the type set forth above. The prepared compounds do not transfer acyl to Coenzyme A.

Chemical Abstracts, Vol. 64, 1966, Abstract No. 1970F. Marly "Carnitineamide derivatives", Belg. 659 194, published August 3, 1965, Chemical Abstracts, Vol. 64, 1960, Abstract No. 11314e, Marly "Carnitineamide d-camphorate", Belg. 660 039, published August 23, 1965, and chemical Abstracts, Vol. 59, 1963, No. 11660 g, Takeda "Carnitineamide", Japan 23, published January 9, 1963 disclose carnitineamides. These have the same hydroxy at the 3-position as carnitine and thus functions to transfer acyl the same way as carnitine. In other words, these compounds cannot competitively inhibit carnitine acyltransferases.

GB—A—1 093 937, JP—A—28 092, FR—A—1 442 318, and US—A—3 185 729 have an amino alpha to the carboxylic acid group while the present compounds have amino beta to the carboxylic acid group. Carnitine acyltransferases function at the beta position and thus are not inhibited by the alpha substituted compounds.

Sire, O., et al., "Carnitine palmitoyltransferase I", European Journal of Biochemistry, *136*, pp. 371—375 (1983) fails to teach any compound analogous to those as prepared in this invention. Sire discloses D-galactosamine which is substantially different in structure. It functions by modifying the membrane and not the enzyme.

Fritz, I. B., et al., "Properties of Partially Purified Carnitine Acetyltransferase", The Jounral of Biological Chemistry, *238*(7), 2509—2517 (7/63) simply teaches substrate acted on by the enzyme carnitine acetyl-transferase. It teaches no compound analogues to the present compounds which inhibits carnitine acetyltransferase.

Chase, J. F. A., et al., "Specific Alkylation of a Histidine Residue in Carnitine Acetyltransferase by Bromoacetyl-L-Carnitine", Biochem. J. 116, 713—720/1970) and Gilbert, R. J., et al., "Bromoacetyl-L-Carnitine: Biochemical and Antitrysanosomal Actions Against Trypanosoma *Brucei Brucei*", Biochemical Pharmacology, *32*(22), 3447—3451 (1983) teach bromoacetyl-L-carnitine. Applicant tried to duplicate the experiment of Gilbert et al. and found that bromoacetyl-L-carnitine functions the same as bromoacetic acid indicating breakdown to bromoacetic acid. In such testing it was found that bromoacetyl-L-carnitine is toxic to the rats tested at 50 mg/kg. These data indicate the instability of this compound in animals contrary to the present compounds.

Fritz, I. B., et al., "Carnitine Acetyltransferase II. Inhibition by Carnitine Analogues and by Sulfhydryl Reagents", The Journal of Biological Chemistry, 240(5), 2188—2192 (5/65) discloses on page 2190 the competitive inhibiting effect of several compounds in respect to carnitine acetyltransferase as indicated by $K'_i$ values. Example II in the application shows the $K'_i$ value for acetylaminocarnitine, i.e., D,L-3-acetamido-4-trimethylaminobutyric acid · $H_2O$. This compound is significantly better than any compound tested by Fritz et al. The best compound of Fritz et al. is No. 3 in the table—D-acetylcarnitine. The analogous amino compound as claimed, D,L-3-acetamido-4-trimethylaminobutyric acid · $H_2O$ is 5 times more effective as shown by the $K'_i$ value and would be 10 times more effective if No. 3 of Fritz et al. were 50% D- and 50% L-. Compound No. 3 of Fritz et al. is known to cause myasthenia gravis to humans.

Kallai-Sanfacon, M. A., et al., "Effect of AY-25,712 and Other Lipid-Lowering Agents on Liver Catalase and Liver Carnitine Acetyltransferase in Rats (41658)", Proceedings of the Society for Experimental Biology and Medicine, *173*, pp. 367—371 (1983) merely discloses the enzyme. They are trying to increase the enzyme effect rather than inhibit it.

Tutwiler G., discussion in *Carnitine Biosynthesis, Metabolism, and Functions,* pp. 171—173, Academic Press, New York, 1980 discloses compounds of much different structure than those prepared in this invention. See the structure of 2-tetradecylglycidic acid on page 173.

Vasudevan, V., et al., "Active-Site-Directed Inhibition of Carnitine Acetyltransferase", Archives of Biochemistry and Biophysics, *220* (1), pp. 193—199 (Jan. 1983) discloses an analogue of coenzyme A rather than an analog of carnitine. Moreover, it would breakdown in an animal.

This invention relates to a process for producing D,L-3-acetamido-4-trimethylaminobutyric acid or salt form or zwitterionic form thereof comprising the steps of reacting 6-(chloromethyl)uracil with dimethylamine, reducing to the dihydrouracil, hydrolyzing to open the ring, acetylating, and then methylating.

This invention relates also to a process for producing D,L-3-amino-4-trimethylaminobutyric acid or salt form or zwitterionic form thereof comprising the steps of reacting 6-(chloromethyl)uracil with dimethylamine, reducing to the dihydrouracil, hydrolyzing to open the ring, acetylating, methylating, then hydrolyzing.

This invention relates also to a process for producing D,L-3-palmitoamido-4-trimethylaminobutyric acid · $H_2O$ comprising the steps of

(a) reacting 6-(chloromethyl)uracil with dimethylamine to form crystalline derivative of 6-(dimethylaminomethyl)uracil,

(b) dissolving the product of step (a) and reducing to form crystalline derivative of 6-(dimethylaminomethyl)dihydrouracil,

(c) dissolving the product of step (b) and hydrolyzing to form crystalline derivative of 3-amino-4-dimethylaminobutyric acid,

(d) dissolving the product of step (c) and acetylating to form crystalline derivative of 3-acetamido-4-dimethylaminobutyric acid,

(e) dissolving the product of step (d) and methylating to form crystalline derivative of 3-acetamido-4-trimethylaminobutyric acid,

(f) dissolving the product of step (e) and hydrolyzing to form crystalline derivative of D,L-3-amino-4-trimethylaminobutyric acid,

(g) acylating with palmitoyl chloride.

This invention relates particularly to the preparation of aminocarnitines having the structural formula:

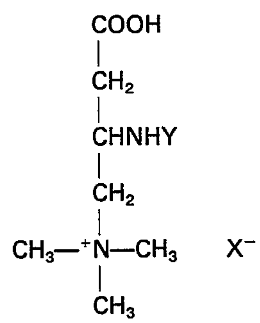

wherein Y is

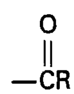

wherein R is an aliphatic group with 9 to 19 carbon atoms, and wherein $X^-$ is a non-toxic counterion; the non-toxic esters and salts thereof; or the zwitterionic form thereof wherein H is removed from the —COOH group and —$COO^-$ serves as the counterion $X^-$.

Additional compounds herein are the non-toxic esters and salts of the acids described in the above paragraph.

Additional compounds herein are the zwitterionic compounds produced by removing H from the —COOH group whereby the —$COO^-$ serves as the counterion $X^-$.

The compounds herein include hydrates, and in such X′ is provided by $OH^-$ in the water of hydration.

Preferred compounds herein include 3-acetamido-4-trimethylaminobutyric acid · $H_2O$ (which may be referred to as acetyl amino carnitine and is hereinafter sometimes referred to as AAC) and 3-amino-4-trimethylaminobutyric acid · $HCl_2$ (which may be referred to as amino carnitine and is hereinafter sometimes referred to as AC).

Usually the D,L form is synthesized herein. If more potency is desired for a given weight, the L-isomer can be isolated by resolution, e.g. using alkaloid salts.

Best mode for carrying out the invention

We turn now in more detail to the description of the amino carnitines herein.

Turning firstly to the acylated and haloacylated amino compounds herein, R and R′ are selected so that the acyl chain length ranges up to 20 carbon atoms and can be saturated or unsaturated including, for example, one, two, three or even four double bonds. The acyl chains and the acyl portion of the haloacylated compounds include, for example, acetyl, propionyl, butyroyl, caproyl, capryloyl, decanoyl, tridecanoyl, lauroyl, myristoyl, myristoleoyl, palmitoyl, stearoyl, oleoyl, linoleoyl, linolenoyl, eleostearoyl, arachidoyl, gadoleoyl, and arachidonoyl.

Turning now to the counterions $X^-$ in the description of the above set forth structure, these are uncritical as long as they are non-toxic since they become separated in solution or in a mammal and can be, for example, hydroxide (such as in water of hydration), chloride, acetate, propionate, phosphate, sulfate, methosulfate, ethosulfate, bicarbonate and carbonate. As indicated above the compounds herein can be in the zwitterionic form wherein hydrogen is removed from the acid group and the resulting $COO^-$ serves as a counterion in place of $X^-$.

Turning now to the embodiment which is in the ester or salt form instead of the acid form, the particular ester group or salt cation is uncritical as long as it is non-toxic since these break down to the acid form in solution or in the body. Thus methyl or ethyl or sodium, for example, are readily substituted for hydrogen in the COOH group.

The acyl and haloacyl amino derivatives are conveniently prepared by starting with the free amino compound and acylating with acid chloride or acid anhydride for the acyl compounds and with haloacyl anhydride for the haloacyl compounds (e.g. bromoacetic anhydride for bromoacyl compounds).

Acetyl amino compound is also readily prepared by reacting 6-(chloromethyl)uracil with dimethylamine, reducing to the dihydrouracil, hydrolyzing to open the ring, acetylating, and then methylating to form the trimethylammonium salt. An alternative route to acetyl amino compound involves reducing uracil-4-acetic acid using hydrogen and rhodium on alumina, hydrolyzing with water and HCl to open the ring, acylating and cyclizing with acetic anhydride, opening the ring with ammonia, decarboxylating in a Hoffman reaction with NaOBr, and then methylating with $CH_3I$ to form the quaternary ammonium salt. A second alternative route involves starting with D- or L-aspartic acid, esterifying the β-carboxyl group with benzyl alcohol, protecting the amino group, e.g. with benzyloxycarbonyl, tert-butyloxycarbonyl or acetyl groups, forming the α-dimethylamide using dicyclohexylcarboimide and dimethylamine, selectively reducing at the 1-position of the carbon chain with tetrabutyl ammonium borohydride, and methylating using $CH_3I$ to form the quaternary ammonium salt. This procedure yields the L- or D-amino carnitine isomer directly.

Free amino compound is readily formed by hydrolyzing the acetyl amino compound. Alternatively, it is prepared by starting with ethyl 4-bromocrotonate, reacting with trimethylamine to form the quaternary ammonium salt, reacting with ammonia to form amine ($NH_2$) and hydrolyzing to remove ethyl and form the acid. In the description of the above set forth structural formula the free amino compound is described both where Y is —H and where Y is $—H_2^+Z^-$.

The following specific examples are illustrative of the invention.

In the examples, temperatures are in the °C.

Example I

D,L-3-acetamido-4-trimethylaminobutyric acid · $H_2O$, i.e. ACC, was prepared as follows:

Dimethylamine (170 ml of a 40% solution in water, 1.5 moles) and 330 ml of water were mixed by magnetic stirring in a 1 ligr Erlenmeyer flask. 6-(chloromethyl)uracil (80.3 gm, 0.5 moles) was added to that mixture in portions over a 15 min. period; the reaction was slighly exothermic. The mixture was stirred until it became clear and then for an additional 30 min. The solution was then heated in a boiling water bath and filtered through a steam-heated filter to remove a small amount of insoluble impurities. The clear filtrate was rotary evaporated at reduced pressure to yield a white solid. Water (200 ml) was twice added to the solid and evaporated to completely remove unreacted dimethylamine. The solid was then suspended in 6 M HCl (500 ml) and the mixture was swirled in a boiling water bath until a clear solution resulted, That solution was rotary evaporated at reduced pressure to a dry solid and water was added and removed twice as described above. The solid was then dissolved in the minimum volume of hot water and allowed to crystallize as the solution cooled. The crystals were collected by filtration, washed with cold 50% aqueous ethanol and then ether, and dried in a vacuum desiccator over $P_2O_5$. The product, 6-(dimethylamino-methyl)uracil · HCl, was obtained as a white solid; mp 282°C; $C_7ClN_3O_2$ requires C: 40.88%, H: 5.88%; N: 20.43%; found C: 40.88, H: 5.66, N: 20.19).

6-(dimethylaminomethyl)uracil · HCl (10.3 gm, 0.05 moles) and 250 ml of 10% acetic acid in water were placed in a 500 ml Parr bottle. The bottle was flushed briefly in a 500 ml Parr bottle. The bottle was flushed briefly with $N_2$ and then 0.5 gm of 5% rhodium on alumina catalyst powder was added. The bottle was attached to a Parr shaking hydrogenator and, after flushing twice with $H_2$, was pressurized to 40 PSI with $H_2$. Hydrogenation was carried out with shaking at room temperature for 24 hours by which time approximately 0.05 mole of $H_2$ had been absorbed. The bottle was then flushed with $N_2$, removed from the hydrogenator and the solution filtered under $N_2$ through a bed of Celite. The filtrate was rotary evaporated under reduced pressure to a white solid which was recrystallized from 50 ml of hot ethanol to which a few ml of water had been added to achieve nearly complete solubility. The crystals were collected by filtration, washed with 5% aqueous ethanol and then ether, and dried in a vacuum desiccator over $P_2O_5$. The product, 6-(dimethylaminomethyl)dihydrouracil · HCl, was obtained as a white solid (yield: 9.3 gm, 90%); mp: 257—258°C.; $C_7H_{14}ClN_3O_2$ requires C: 40.49%; H: 6.80%; N: 20.24%; found C: 40.67%; H: 6.79%; N: 19.95%).

6-(dimethylaminomethyl)dihydrouracil · HCl (10.4 gm, 0.05 moles) was dissolved in 300 ml of 6N HCl in a 500 ml round bottom flask. A condenser was fitted and the solution was refluxed for 30 hours using a heating mantle. The solution was then cooled and rotary evaporated to dryness at reduced pressure to yield a white solid. That material was dissolved in 10 ml of water and the resulting solution was applied to the top of a column (2.5×45 cm) of Dowex 50×8 ($H^+$ form, 200—400 mesh). The column was developed using a linear gradient formed between 1N HCl and 6N HCl (total volume 1600 ml); the gradient was followed with 200 ml of 6N HCl. Fractions of approximately 25 ml were collected; a 10 μl portion of every other fraction was assayed with o-phthalaldehyde to determine where compounds with primary amino groups eluted. 3-amino-4-dimethylaminobutyric acid · $HCl_2$ eluted at about 4.5 HCl and was the major o-phthalaldehyde-positive species detected. The appropriate fractions were pooled and rotary evaporated to dryness at reduced pressure. Water was added and removed twice to ensure that no free HCl remained in the product. The resulting wite solid, 3-amino-4-dimethylaminobutyric acid · $HCl_2$, was pure without

recrystallization (yield 9.2 gm, 84%); mp 220—221°; $C_6H_{16}Cl_2N_2O_2$ requires C: 32.89%; H: 7.36%; N: 12.79%; found C: 33.04%; H: 7.40%; N: 12.56%).

3-amino-4-dimethylaminobutyric acid · $HCl_2$ (10.96 gm, 0.05 moles) was dissolved in 300 ml of 0.5 N NaOH (the pH was 10—11) in a 500 ml Erlenmeyer flask. The solution was chilled to <5° and sodium carbonate (12.4 gm, 0.1 mole) was added. With vigorous magnetic stirring, acetic anhydride (14.8 ml; 150 mmole) was added dropwise over a 15 minute period. After stirring an additional 30 minutes at 0—5°, an aliquot was assayed with o-phthalaldehyde to confirm that no free primary amino groups remained. The mixture was then cautiously acidified to pH 2 with concentrated HCl ($CO_2$ evolution observed) and the resulting solution was rotary evaporated at reduced pressure to a gummy solid. That material was suspended in 100 ml of concentrated HCl and that mixture was filtered to remove NaCl. The filtrate was rotary evaporated under reduced pressure to yield a white amorphous solid which was not generally further purified. For purposes of characterization, the crude product was chromatographed on Dowex 50 ($H^+$) using the procedure described for 3-amino-4-dimethylaminobutyric acid · $HCl_2$. The acetylated product, located by monitoring $A_{210}$, eluted at about 2.5 M HCl. Appropriate fractions were pooled, and rotary evaporated at reduced pressure to yield a product contaminated with NaCl. The crude material was dissolved in 25 ml of water and that solution was applied to a column (2.5×20 cm) of Dowex 50×8 ($H^+$, 200—400 mesh). The column was washed with 1000 ml of water and the product was then eluted with 3N $NH_4OH$. Fractions containing product were washed with $H_2O$ and rotary evaporated to dryness under reduced pressure to yield a white solid. The resulting solid, 3-acetamido-4-dimethylaminobutyric acid · $H_2O$, was pure without recrystallization (yield: 8.54 gm, 83%); mp 107—110°; $C_8H_{16}N_2O_3 \cdot H_2O$ requires C: 46.59%; H: 8.80%; N: 13.58%; found C: 46.96%; H: 8.86%; N: 13.85%).

3-acetamido-4-dimethylaminobutyric acid · $H_2O$ (11.2 gm of unpurified material, 0.05 moles) was dissolved in 75 ml of water in a large screw cap bottle and the resulting solution was adjusted to pH 7 with 10 N NaOH. Sodium carbonate (12.4 gm, 0.1 moles), methanol (75 mls) and iodomethane (6.2 mls, 0.1 mole) were then added and the bottle was capped and stirred magnetically at 25° for 24 hours. The solution was then diluted with 250 ml of water and acidified to pH 2 with concentrated HCl. The resulting solution was rotary evaporated under reduced pressure to yield a gummy yellow solid which was suspended in 50 ml of concentrated HCl. That solution was filtered to remove NaCl, and the filtrate was rotary evaporated under reduced pressure to yield a yellow gum. The residue was dissolved in 20 ml of water and chromatographed on Dowex 50 ($H^+$) as described for 3-amino-4-dimethylaminobutyric acid · $HCl_2$. The product, 3-acetamido-4-trimethylaminobutyric acid · $H_2O$, located by monitoring the $A_{210}$ of the fractions, was eluted at about 3N HCl. The appropriate fractions were pooled and rotary evaporated at reduced pressure to yield a white solid contaminated with a small amount of NaCl. That material was dissolved in water and absorbed to a column (2.5×20 cm) of Dowex 50 ($H^+$). After washing with 1000 ml of water, the product was eluted with 750 ml of 3N $NH_4OH$ and the appropriate fractions were rotary evaporated to dryness under reduced pressure. The resulting white solid D,L-3-acetamido-4-trimethylaminobutyric acid ·$H_2O$, was pure without recrystallization (yield 9.7 gm); mp 202—202.5° $C_9H_{18}N_2O_3 \cdot H_2O$ requires C: 49.07%; H: 9.15%; N: 12.72%; found C: 49.01%; H: 8.94%; N: 12.53%).

Example II

D,L-3-acetamido-4-trimethylaminobutyric acid · $H_2O$, referred to hereinafter as AAC, was assayed for inhibition of carnitine acetyltransferase.

The procedure used is described in Fritz, I. B., Schultz, S. K., and Srere, P. A., "Properties of Partially Purified Carnitine Acetyltransferase", J. Biol. Chem., *238* 2509—2517 (1963).

The assay was based on the following reactions wherein CAT is used to denote carnitine acetyltransferase, CS is used to denote citrate synthase, MDH is used to denote malate dehydrogenase, NAD is used to denote β-nicotinamide adenine dinucleotide (unreduced form) and NADH is used to denote β-nicotinamide adenine dinucleotide (reduced form):

| Catalyst | Reaction |
|---|---|
| (1) CAT | Acetylcarnitine+Coenzyme A⇄ carnitine+acetyl-Coenzyme A |
| (2) CS | Acetyl-Coenzyme A+oxaloatetate→ citrate+Coenzyme A |
| (3) MDH | Malate+NAD⇄oxaloacetate+ NADH+$H^+$ |

The assay is based on the fact that the velocity of reaction to produce acetyl-Coenzyme A is reduced to the extent that CAT is inhibited. Reactions (2) and (3) are a detection system for acetyl-Coenzyme A wherein acetyl-Coenzyme A, as it is produced, reacts with oxaloacetate thereby causing the MDH to catalyze production of oxaloacetate to replace that which is used and resulting in production of NADH which is detected using a spectrophotometer based on its property of absorbing light at 340 mμ. Thus, when CAT is bound and inhibited, the rate of NADH production is decreased and the rate of increase of absorbency at 340 mμ is decreased. The results are readily compiled in a Lineweaver-Burk plot of the reciprocal of

reaction velocity versus the reciprocal of concentration of acetylcarnitine and the data can be analyzed to provide $K_i'$ values as mentioned above.

Sixteen runs were carried out. They consisted of a set of 4 runs where 1 mM of D,L-acetylcarnitine (pH 8.0) was included, a set of 4 runs where 2 mM of D,L-acetylcarnitine was included, a set of 4 runs where 5 mM of D,L-acetylcarnitine was included and a set of 4 runs where 10 mM of D,L-acetylcarnitine was included. In each set of runs, one run was conducted without AAC, one run was conducted with 0.1 mM AAC, one run was conducted with 0.5 mM AAC, and one run was conducted with 1.0 mM AAC.

Each run was carried out utilizing a cuvette with 1 ml total being added to it. To each cuvette was added 0.7 ml of a premix of aqueous solutions of Tris-HCl buffer, $NAD^+$, dithiothreitol, and Tris D,L-malate, pH of 8.0 (concentrations in the premix were as follows: Tris-HCl, 143 mM; $NAD^+$, 0.71 mM; dithiothreitol, 4.3 mM; and Tris D,L-malate, 14.3 mM, the selected amount of D,L-acetylcarnitine as described above, 0.05 ml of a 4 mM aqueous solution of Coenzyme A, 0.025 ml of a 168 units/ml solution of CS, 0.025 ml of a 240 units/ml solution of MDH, the selected amount of AAC as described above, and water to 0.995 ml. The D,L-acetylcarnitine was added utilizing solution of concentration of 100 mM (in other words 0.010 ml for the first set of runs, 0.020 ml for the second set of runs, 0.050 ml for the third set of runs, 0.100 ml for the fourth set of runs). The AAC was added utilizing solution of concentration of 100 mM (in other words, additions for runs in each set were of 0 ml, 0.001 ml, 0.005 ml and 0.010 ml).

After the above described reactants were added, each cuvette and its contents were incubated for 15 minutes at 30°C.

Directly after the incubation period, 0.005 ml of CAT (concentration 5 units/ml) was added in each case.

The reaction rate in each case was measured as described above as a function of change in absorbance at 340 mμ (providing a time course of NADH formation) and was expressed as μmole NADH/min. (1 mM $NADH=6.2\times\Delta A_{340}$).

A Lineweaver-Burk plot was then prepared and all the lines passed through a point on the Y-axis in common with the control (no ACC) proving binding to the same site as acetyl carnitine and proving AAC is a competitive inhibitor of CAT binding it in a 1:1 molar ratio. A $K_i'$ value for the D,L form of AAC was calculated to be $5\times10^{-5}$M which is $10^{-2}$ to $10^{-3}$ times the $K_i'$ values found by Fritz and Schultz for the compounds they tested. The AAC was found to inhibit formation of acetyl-Coenzyme A as follows: at a concentration of 0.1 mM, 34%; at a concentration of 0.5 mM, 67%; at a concentration of 1 mM, 81%. The plot indicated that AAC binds to CAT 18 times more tightly than acetylcarnitine. The binding action was found to be reversible by dilution.

Example III

An example of the research utility of AAC is set forth below.

Four mice were injected subcutaneously with 0.5 mmol/kg carbon 14 tagged acetylcarnitine. Two of them were injected intra peritoneally with 5 mmol/kg of AAC. The carbon 14 $CO_2$ output of the mice was monitored every 15 minutes for 6 hours and for each mouse a graph was prepared with time on the X-axis and % of radiolabel $CO_2$ on the Y-axis. The data indicated a retarded rate of radiolabel $CO_2$ output for the mice injected with AAC indicating that AAC is active *in vivo* in inhibiting CAT and that CAT activity plays a major role in the catabolism of acetylcarnitine *in vivo*. The amount of AAC administered was not toxic to the mice.

Example IV

3-Acetamide-4-trimethylaminobutyric acid · $H_2O$ (3.3 gm, 0.15 moles) prepared as set forth in Example I was dissolved in 90 mls of 2N HCl and the resulting solution was refluxed for 6 hours. The solution was then cooled and rotary evaporated at reduced pressure to yield a white solid. That material was dissolved in 20 ml of water and chromatographed on Dowex 50 ($H^+$) as described in Example I. The product (located by assay of fraction aliquots using o-phthalaldehyde eluted at about 4N HCl. The appropriate fractions were pooled, and rotary evaporated under reduced pressure to yield a white solid which analysis indicated to be D,L-3-amino-4-trimethylaminobutyric acid · $HCl_2$ (i.e. AC) (yield 2.7 gm, 77%); mp 214—217°; $C_7H_{18}Cl_2N_2O_2$ requires C: 36.06%; H: 7.78%; N: 12.02%; found C: 35.99%; H: 7.79%; N: 11.87%.

The AC is readily converted to D,L-3-palmitoamido-4-trimethylaminobutyric acid · $H_2O$ (i.e. PAC) by acylating with palmitoyl chloride. The PAC readily binds to and inhibits carnitine palmitoyltransferase and is useful to investigate the role of fatty acid catabolism in diabetes. Such investigation is readily carried out, e.g. by utilizing mice with drug induced diabetes and intra peritoneally injecting some of these mice with, e.g. 0.1—50 mmol/kg of PAC and observing whether the mice treated wih PAC are less ketotic than the other mice.

The AC is readily converted to D,L-3-bromoacetamido-4-trimethylaminobutyric acid · $H_2O$ by acylating with bromoacetic anhydride. The formed compound inhibits CAT similarly to AAC but the binding action is irreversible.

The term "non-toxic counterion" is used herein in its conventional sense as meaning that the counterion is non-toxic in the amounts that would be present in association with the administration of the compounds in useful amounts.

While the foregoing describes preferred embodiments, modifications within the scope of the invention

will be evident to those skilled in the art. Thus, the scope of the invention is intended to be defined by the claims.

**Claims**

1. A process for producing D,L-3-acetamido-4-trimethylaminobutyric acid or salt form or zwitterionic form thereof comprising the steps of reacting 6-(chloromethyl)uracil with dimethylamine, reducing the dihydrouracil, hydrolyzing to open the ring, acetylating, and then methylating.

2. A process as recited in Claim 1 for producing D,L-3-acetamido-4-trimethylaminobutyric acid · $H_2O$ comprising the steps of

(a) reacting 6-(chloromethyl)uracil with dimethylamine to form crystalline derivative of 6-(dimethylaminomethyl)uracil,

(b) dissolving the product of step (a) and reducing to form crystalline derivative of 6-(dimethylaminomethyl)dihydrouracil,

(c) dissolving the product of step (b) and hydrolyzing to form crystalline derivative of 3-amino-4-dimethylaminobutyric acid,

(d) dissolving the product of step (c) and acetylating to form crystalline derivative of 3-acetamido-4-dimethylaminobutyric acid,

(e) dissolving the product of step (d) and methylating and recovering crystalline product.

3. A process for producing D,L-3-amino-4-trimethylaminobutyric acid or salt form or zwitterionic form thereof comprising the steps of reacting 6-(chloromethyl)uracil with dimethylamine, reducing to the dihydrouracil, hydrolyzing to open the ring, acetylating, methylating, then hydrolyzing.

4. A process as recited in Claim 3 for producing D,L-3-amino-4-trimethylaminobutyric acid · $HCl_2$ comprising the steps of

(a) reacting 6-(chloromethyl)uracil with dimethylamino to form crystalline derivative of 6-(dimethylaminomethyl)uracil,

(b) dissolving the product of step (a) and reducing to form crystalline derivative of 6-(dimethylaminomethyl)--dihydrouracil,

(c) dissolving the product of step (b) and hydrolyzing to form crystalline derivative of 3-amino-4-dimethylaminobutyric acid,

(d) dissolving the product of step (c) and acetylating to form crystalline derivative of 3-acetamido-4-dimethylaminobutyric acid,

(e) dissolving the product of step (d) and methylating to form crystalline derivative of 3-acetamido-4-trimethylaminobutyric acid,

(f) dissolving the product of step (e) and hydrolyzing and recovering crystalline product.

5. A process for producing D,L-3-palmitoamido-4-trimethylaminobutyric acid · $H_2O$ comprising the steps of

(a) reacting 6-(chloromethyl)uracil with dimethylamine to form crystalline derivatives of 6-(dimethylaminomethyl)uracil,

(b) dissolving the product of step (a) and reducing to form crystalline derivative of 6-(dimethylaminomethyl)dihydrouracil,

(c) dissolving the product of step (b) and hydrolyzing to form crystalline derivate of 3-amino-4-dimethylaminobutyric acid,

(d) dissolving the product of step (c) and acetylating to form crystalline derivative of 3-acetamido-4-dimethylaminobutyric acid,

(e) dissolving the product of step (d) and methylating to form crystalline derivative of 3-acetamido-4-trimethylaminobutyric acid,

(f) dissolving the product of step (e) and hydrolyzing to form crystalline derivatives of D,L-3-amino-4-trimethylaminobutyric acid,

(g) acylating with palmitoyl chloride.

6. A process as recited in Claim 1 wherein the hydrolyzing to open the ring is an acid hydrolysis.

7. A process as recited in Claim 3 wherein the hydrolyzing to open the ring is an acid hydrolysis.

8. A process as recited in Claim 5 wherein the hydrolyzing in step (c) is an acid hydrolysis.

**Patentansprüche**

1. Ein Verfahren zur Herstellung von D,L-3-Acetamido-4-trimethylaminobuttersäure oder der Salzform oder der zwitterionischen Form davon, dadurch gekennzeichnet, daß die Stufen Reaktion von 6-(Chloromethyl)uracil mit Dimethylamin, Reduktion zu Dihydrouracil, Hydrolyse, um den Ring zu öffnen, Acetylierung und dann Methylierung durchgeführt werden.

2. Verfahren zur Herstellung von D,L-3-Acetamido-4-trimethylaminobuttersäure · $H_2O$ nach Anspruch 1, dadurch gekennzeichnet, daß die Stufen

(a) Reaktion von 6-(Chlormethyl)uracil mit Dimethylamin um das kristalline Derivat des 6-(Dimethylaminomethyl)uracils zu bilden,

(b) Auflösung des Produkts nach Stufe (a) und Reduktion, um das kristalline Derivat des 6-(Dimethylaminomethyl)dihydrouracils zu bilden,

(c) Auflösung des Produkts nach Stufe (b) und Hydrolyse, um das kristalline Derivat der 3-Amino-4-dimethylaminobuttersäure zu bilden,

(d) Auflösung des Produkts nach Stufe (c) und Acetylierung, um das kristalline Derivat der 3-Acetamido-4-dimethylaminobuttersäure zu bilden,

(e) Auflösung des Produkts nach Stufe (d) und Methylierung und Wiedergewinnung des kristallinen Produkts durchgeführt werden.

3. Verfahren zur Herstellung von D,L-3-Amino-4-trimethylaminobuttersäure oder der Salzform oder der zwitterionischen Form davon, gekennzeichnet dadurch, daß die Stufen Reaktion von 6-(Chlormethyl)uracil mit Dimethylamin, Reduktion zum Dihydrouracil, Hydrolyse, un den Ring zu öffnen, Acetylierung, Methylierung und dann Hydrolyse durchgeführt werden.

4. Verfahren zur Herstellung von D,L-3-Amino-4-trimethylaminobuttersäure · $HCl_2$ nach Anspruch 3, dadurch gekennzeichnet, daß die Stufen

(a) Reaktion von 6-(Chloromethyl)uracil mit Dimethylamin, um das kristalline Derivat des 6-(Dimethylaminomethyl)uracils zu bilden,

(b) Auflösung des Produkts nach Stufe (a) und Reduktion, um das kristalline Derivat des 6-(Dimethylaminomethyl)dihydrouracils zu bilden,

(c) Auflösung des Produkts nach Stufe (b) und Hydrolyse, um das kristalline Derivat der 3-Amino-4-dimethylaminobuttersäure zu bilden,

(d) Auflösung des Produkts nach Stufe (c) und Acetylierung, um das kristalline Derivat der 3-Acetamido-4-dimethylaminobuttersäure zu bilden,

(e) Auflösung des Produkts nach Stufe (d) und Methylierung, um das kristalline Derivat der 3-Acetamido-4-trimethylaminobuttersäure zu bilden,

(f) Auflösung des Produkts nach Stufe (e) und Hydrolyse und Gewinning des kristallinen Produkts durchgeführt werden.

5. Verfahren zur Herstellung von D,L-3-Palmitoamido-4-trimethylaminobuttersäure · $H_2O$, dadurch gekennzeichnet, daß die Stufen

(a) Reaktion von 6-(Chlormethyl)uracil mit Dimethylamin, um das kristalline Derivat des 6-(Dimethylaminomethyl)uracils zu bilden,

(b) Auflösung des Produkts nach Stufe (a) und Reduktion, um das kristalline Derivat des 6-(Dimethylaminomethyl)dihydrouracils zu bilden,

(c) Auflösung des Produkts nach Stufe (b) und Hydrolyse, um das kristalline Derivat der 3-Amino-4-dimethylaminobuttersäure zu bilden,

(d) Auflösung des Produkts nach Stufe (c) und Acetylierung, um das kristalline Derivat der 3-Acetamido-4-dimethylaminobuttersäure zu bilden,

(e) Auflösung des Produkts nach Stufe (d) und Methylierung, um das kristalline Derivat der 3-Acetamido-4-trimethylaminobuttersäure zu bilden,

(f) Auflösung des Produkts nach Stufe (e) und Hydrolyse, um das kristalline Derivat der D,L-3-Amino-4-trimethylaminobuttersäure zu bilden,

(g) Acylierung mit Palmitoylchlorid durchgeführt werden.

6. Verfahren nach Anspruch 1, dadurch gekennzeichnet, daß die Hydrolyse zur Ringöffnung eine saure Hydrolyse ist.

7. Verfahren nach Anspruch 3, dadurch gekennzeichnet, daß die Hydrolyse zur Ringöffnung eine saure Hydrolyse ist.

8. Verfahren nach Anspruch 5, dadurch gekennzeichnet, daß die Hydrolyse in Stufe (c) eine saure Hydrolyse ist.

**Revendications**

1. Procédé pour produire l'acide D,L-3-acétamido-4-triméthyl-aminobutyrique ou une forme salimone zwittérionique de celui-ci, caractérisé en ce qu'il comprend les étapes de réaction du 6-(chlorométhyl) uracile avec de la diméthylamine, de réduction en dihydrouracile, d'hydrolyse pour ouvrir le cycle, d'acétylation, puis de méthylation.

2. Procédé suivant la revendication 1, pour produire l'acide D,L-3-acétamido-4-triméthylaminobutyrique, $H_2O$, caractérisé en ce qu'il comprend les étapes suivantes:

a) la réaction du -(chlorométhyl) uracile avec de la dimèthylamine pour former un dérivé cristallin du 6-(diméthylamino-méthyl)uracile.

b) la dissolution du produit de l'étape (a) et la réduction pour former un dérivé cristallin du 6-(diméthylaminométhyl)-dihydrouracile.

c) la dissolution du produit de l'étape b) et l'hydrolyse pour former un dérivé cristallin de l'acide 3-amino-4-diméthylaminobutyrique.

d) la dissolution de produit de l'étape (c) et l'acétylation pour former un dérivé cristallin de l'acide 3-acétamido-4-diméthylaminobutyrique.

e) la dissolution du produit à l'étape d) et la méthylation, puis la récupération du produit cristallin.

3. Procédé pour produire l'acide D,L-3-amino-4-triméthylamino-butyrique, ou une forme saline ou zwittérionique, de celui-ci, caractérisé en ce qu'il comprend les étapes de réaction du 6-(chlorométhyl) uracile avec la diméthylamine, de réduction en dihydrouracile, d'hydrolyse pour ouvrir le cycle, d'acétylation, de méthylation, puis d'hydrolyse.

4. Procédé suivant la revendication 3, pour produire l'acide D,L-3-amino-4-trimethylaminobutyrique $HCl_2$, caractérisé en ce qu'il comrpend les étapes suivantes:

a) la réaction du 6-(chlorométhyl) uracile avec de la diméthylamine pour former un dérivé cristallin du 6-(diméthylamino-méthyl)uracil,

b) la dissolution du produit de l'étape a) et la réduction pour former un dérivé cristallin du 6-(diméthylaminométhyl)-dihydrouracile.

c) la dissolution du produit de l'étape b) et l'hydrolyse pour former le dérivé cristallin de l'acide 3-amino-4-diméthylaminobutyrique.

d) la dissolution du produit de l'étape (c) et l'acithylation pour former un dérivé cristallin de l'acide 3-acetamido-4-dimethylaminobutyrique.

e) la dissolution du produit de l'étape d) et la méthylation pour former un dérivé cristallin de l'acide 3-acétamido-4-dimethylaminobutyrique.

f) la dissolution du produit de l'étape (e) et l'hydrolyse, puis la récupération du produit cristallin.

5. Procédé pour produire l'acide D,L-3-palmitoamido-4-triméthylaminobutyrique $H_2O$ caractérisé en ce qu'il comprend les étapes suivantes:

a) la réaction de 6-(chlorométhyl)uracil avec de la diméthylamine pour former un dérivé cristallin du 6-(diméthylaminométhyl)uracile.

b) la dissolution du produt de l'étape (a) et la réduction pour former un dérivé cristallin du 6-(dimethylaminométhyl)-dihydrouracile.

c) la dissolution du produit de l'étape (b) et l'hydrolyse pour former un dérivé cristallin de l'acide 3-amino-4-diméthylaminobutyrique.

d) la dissolution du produit de l'étape (c) en l'acétylation pour former un dérivé cristallin de l'acide 3-acetamido-4-diméthylaminobutyrique.

e) la dissolution du produit de l'étape (d) et la méthylation pour former un dérivé cristallin de l'acide 3-acetamido-4-triméthylaminobutyrique.

f) la dissolution du produit de l'étape (e) et l'hydrolyse pour former un dérivé cristallin de l'acide D,L-3-amino-4-triméthylaminobutyrique.

g) l'acylation avec le chlorure de palmitoyle.

6. Procédé suivant la revendication 1, caractérisé en ce que l'hydrolyse effectuée pour ouvrir le cycle est une hydrolyse acide.

7. Procédé suivant la revendication 3, caractérisé en ce que l'hydrolyse effectuée pour ouvrir le cycle est une hydrolyse acide.

8. Procédé suivant la revendication 5, caractérisé en ce que l'hydrolyse effectuée dans l'étape (c) est une hydrolyse acide.